# EUROPÄISCHE PATENTSCHRIFT

(11) **EP 1 433 777 B1**
(45) Veröffentlichungstag und Bekanntmachung des Hinweises auf die Patenterteilung: **10.03.2010**
(21) Anmeldenummer: 03025637.4
(22) Anmeldetag: 06.11.2003
(51) Int. Cl.: C07C 249/04

(54) **Verfahren zur Herstellung von Oximen**
Process for the preparation of oximes
Procédé pour la préparation d'oximes

(30) Priorität: 23.12.2002 DE 10260717
(43) Veröffentlichungstag der Anmeldung: 30.06.2004
(73) Patentinhaber: Evonik Degussa GmbH, 45128 Essen (DE)
(72) Erfinder: Leininger, Stefan, Dr., 63452 Hanau (DE); Herwig, Jürgen, Dr., 46569 Hünxe (DE); Roos, Martin, Dr., 45721 Haltern (DE); Oenbrink, Georg, Dr., 48249 Dülmen (DE)

(56) Entgegenhaltungen:
- EP-A- 0 496 385
- DE-A- 10 142 620
- GB-A- 1 113 619

## Beschreibung

Die Erfindung betrifft ein verbessertes Verfahren zur Herstellung von Oximen durch Ammoximierung von Carbonylverbindungen mit 6 bis 20 C-Atomen, insbesondere Ketonen mit 8 bis 12 C-Atomen. Das erfindungsgemäße Verfahren betrifft insbesondere die Aufarbeitung des Reaktionsgemisches aus der Ammoximierung und Abtrennung von Wasser zwecks Rezyklierung des organischen Lösungsmittels.

Die Patentanmeldungen EP 0 208 311, EP 0 496,385 EP 0 299 430, EP 0 564 040 und die US-Patentschrift 4,745,221 lehren die Ammoximierung von Carbonylverbindungen, wie inbesondere Alkanonen und Cycloalkanonen, mit Wasserstoffperoxid und Ammoniak in Gegenwart eines aus den Elementen Silicium, Titan und Sauerstoff aufgebauten heterogenen Katalysators. Vollständige Umsätze und damit verbunden eine einfache Aufarbeitung und Rückgewinnung des verwendeten organischen Lösungsmittels werden nur für die Ammoximierung von Cyclohexanon beschrieben. Bekanntlich ist Cyclohexanonoxim Rohstoff für das durch Beckmann-Umlagerung hergestellte Caprolactam.

Bei der Ammoximierung von sterisch anspruchsvolleren Ketonen, wie Alkanonen oder Cycloalkanonen mit mehr als 6 Kohlenstoffatomen, sind die Ausbeuten wegen der geringeren Reaktionsgeschwindigkeiten und niedrigeren Wasserstoffperoxid-Selektivitäten generell niedriger. Zur Vermeidung dieser Nachteile wurde vorgeschlagen, das Katalysatorsystem um weitere Komponenten, im weiteren als Cokatalysatoren bezeichnet, zu ergänzen. So werden in der DE 195 21 011 amorphe Silikate, in der DE 100 47 435 saure Feststoffe und in der DE 101 03 581 Ammoniumionen als Cokatalysatoren beschrieben.

Wie in den Offenlegungsschriften DE 100 47 435 und DE 101 03 581 beschrieben, verläuft die Ammoximierung von großen und sterisch abgeschirmten Alkanonen und Cycloalkanonen dann genügend rasch und selektiv, wenn die Umsetzung in Gegenwart eines Suspensionskatalysators und in Gegenwart eines mit Wasser vollständig oder teilweise mischbaren organischen Lösungsmittels, insbesondere eines kurzkettigen Alkohols mit 1 bis 6 Kohlenstoffatomen, durchgeführt wird.

Bei der beschriebenen Ammoximierung reagiert das eingesetzte Alkanon oder Cycloalkanon mit Ammoniak und Wasserstoffperoxid am Titansilikalit zu dem korrespondierenden Oxim.

Zusätzlich zur Ammoximierung können durch Folgeoxidationen von in einer Parallelreaktion gebildetem Hydroxylamin mit Wasserstoffperoxid Dihydroxylamin, Nitrosyl, salpetrige Säure, Salpetersäure sowie Distickstoffmonoxid als die Peroxidselektivität mindernde Nebenprodukte gebildet werden - siehe US 4,745,221. Es wurde ferner festgestellt, dass mit zunehmenden Umsätzen von Alkanonen und Cycloalkanonen die Peroxidselektivität, bezogen auf das gebildete Oxim, abnimmt und sich vollständige Umsätze nur bei gleichzeitig erhöhter Bildung von Nebenprodukten realisieren lassen.

Während der Umsetzung steigt folglich der Gehalt an Wasser in der Reaktionsmischung an. Da die Löslichkeit von längerkettigen und/oder sterisch anspruchsvolleren Alkanonen und Cycloalkanonen, wie zum Beispiel Cyclooctanon und Cyclododecanon, sowie deren entsprechenden Oximen mit steigendem Wassergehalt im Reaktionsgemisch stark abnimmt, ist es zweckmäßig, die Menge an Wasser in der Ammoximierung möglichst zu begrenzen. Dies gelingt dadurch, dass Wasserstoffperoxid als möglichst konzentrierte wässrige Lösung und Ammoniak als trockenes Gas eingesetzt werden.

Um die Umsetzung zu verbessern, besteht ein Interesse daran, durch die Auswahl des Katalysators, der allein oder gegebenenfalls in Kombination mit Cokatalysatoren Anwendung finden soll, Wasser erzeugende und die Peroxidselektivität senkende Nebenreaktionen zu minimieren.

An die Ammoximierung schließt sich, wie vielen Dokumenten zu entnehmen ist, eine destillative und/oder extraktive Aufarbeitung der Reaktionsmischung an - siehe zum Beispiel EP 0 496 385, EP 0 208 311, EP 0 690 045, EP 0 735 017 und US 4,794,198.

In einem Prozess mit geschlossenen oder teilweise geschlossenen Lösungsmittelkreisläufen, also bei einer kontinuierlichen Ammoximierung, muss darüber hinaus das eingetragene und während der Reaktion gebildete Wasser in einem integrierten oder separaten Aufarbeitungsschritt abgetrennt werden, um anwesende organische Lösungsmittel wieder in die Ammoximierungsstufe zurückführen zu können.

Ein Nachteil der destillativen Aufarbeitung ist, dass praktisch das gesamte Lösungsmittelgemisch destilliert werden muss, was zu einem hohen Energiebedarf und damit Minderung der Wirtschaftlichkeit des Prozesses führt.

Ein zusätzlicher Nachteil von Reaktionslösungen von Oximen mit höheren Molekulargewichten ist, dass sich die Carbonylverbindung und sein Oxim nicht oder nur unvollständig destillativ voneinander trennen lassen. Demgemäß besteht in solchen Fällen ein hohes Interesse daran, einen möglichst vollständigen Keton-Umsatz zu erzielen, um Oxime mit möglichst niedrigem Carbonylgehalt erhalten zu können.

In der noch nicht publizierten Patentanmeldung DE 101 42 620 wird ein gegenüber einer destillativen Aufarbeitung verbessertes Verfahren zur Abtrennung des Oxims aus den Reaktionsgemisch beschrieben. Das Oxim wird durch Kristallisation aus dem Reaktionsgemisch abgetrennt und die Mutterlauge durch anschließende Pervaporation beziehungsweise Dampfpermeation unter Verwendung mindestens einer Membrantrennstufe zumindest teilweise von Wasser befreit. Nachteilig ist hier, dass im Falle der Oximierung von Ketonen mit höherem Molekulargewicht bei unvollständigen Keton-Umsätzen, die Membranen recht schnell durch Keton-Ablagerungen und/oder durch Ablagerungen von als Cokatalysatoren anwesenden Ammoniumsalzen verstopft werden können, wodurch eine aufwendige Reinigung oder gar ein Membran-Austausch notwendig wird.

Aufgabe der Erfindung ist es demgemäß, ein verbessertes Verfahren zur Herstellung von Oximen, umfassend eine Ammoximierung einer Carbonylverbindung, mit Ammoniak und Wasserstoffperoxid in Gegenwart eines titanhaltigen Katalysators, Isolierung des Oxims aus dem Reaktionsgemisch durch Kristallisation, Ausschleusung von Wasser aus dem System und Rückführung des organischen Lösungsmittels in die Ammoximierung, aufzuzeigen, das die Nachteile einer Membrantrennstufe nicht aufweist. Das verbesserte Verfahren sollte sich in besonderer Weise zur Herstellung von Oximen mit mehr als 6 C-Atomen eignen, und insbesondere cyclischen Oximen mit 8 bis 12 C-Atomen. Die Ammoximierung selbst sollte sich in bekannter Weise durchführen lassen und nicht durch die Maßnahmen zur Verbesserung der Aufarbeitung nachteilig beeinflusst werden.

Es wurde überraschend gefunden, dass sich die genannten Aufgaben lösen lassen, indem speziell ausgewählte Lösungsmittel eingesetzt werden.

Gegenstand der Erfindung ist daher ein Verfahren zur Herstellung von Oximen durch Ammonoximierung von Carbonylverbindungen, umfassend
(i) Umsetzung einer Carbonylverbindung mit 6 bis 20 C-Atomen mit Ammoniak und Wasserstoffperoxid in Gegenwart eines zumindest teilweise wasserlöslichen organischen Lösungsmittels und eines titanhaltigen heterogenen Katalysators,
(ii) Abtrennung des Katalysators vom Reaktionsgemisch,
(iii) Auskristallisation des Oxims und Abtrennung desselben von der Mutterlauge,
(iv) Ausschleusung von Wasser aus der Mutterlauge und
(v) Rückführung des Lösungsmittels in die Stufe (i), das dadurch gekennzeichnet ist, dass man in der Stufe (i) ein unter Ammoximierungsbedingungen stabiles Lösungsmittel verwendet, das einen Siedepunkt größer 100°C aufweist und/oder mit Wasser ein zweiphasiges Azeotrop bildet, aus der Mutterlauge Wasser oder ein wasserhaltiges zweiphasiges Azeotrop abdestilliert und den Destillationssumpf und, sofern erwünscht, die überwiegend organische Phase des Azeotrops in die Stufe (i) zurückführt.

Die Unteransprüche richten sich auf bevorzugte Ausführungsformen des erfindungsgemäßen Verfahrens.

Gemäß einer ersten Alternative werden Lösungsmittel verwendet, deren Siedpunkt größer als 100 °C, insbesondere größer 110 °C ist und besonders bevorzugt im Bereich von 110 bis 200 °C liegt. Unter den Ammoximierungsbedingungen sind insbesondere ein- oder mehrwertige Alkohole geeignet, welche die nötige Wasserlöslichkeit und Stabilität aufweisen. Beispiele sind n- und iso-Amylalkohole und Hexanole, soweit sie kein Azetrop mit Wasser bilden, insbesondere aber Ethylenglykol, 1,2-und 1,3-Propylenglykol, 1,2- und 1,4-Butandiol, 1,5-Pentandiol und 1,6-Hexandiol.. Derartige Diole, im Prinzip sind auch Triole, wie Glycerin, einsetzbar, sind gut handhabbar. Bevorzugt sind die Diole mit primären Hydroxylgruppen, insbesondere Ethylenglycol und 1,3-Propylenglycol.

Gemäß einer zweiten Alternative bilden die Lösungsmittel, insbesondere handelt es sich auch hier um Alkohole, mit Wasser unter 100 °C siedende Azeotrope, welche zweiphasig kondensieren, wobei eine Phase wasserreich (> 50 %) ist. Besonders geeignete Beispiele sind Butanole und Amylalkohole, insbesondere n-Butanol, tert.-Butanol, Isoamylalkohol. n-Butanol ist besonders geeignet, weil das zweiphasige Azeotrop eine leichte Abtrennung von Wasser aus der Mutterlauge ermöglicht.

Die Art der Kristallisation des Oxims aus dem Reaktionsgemisch der Ammoxmimierung lässt sich in gleicher Weise durchführen, wie in der DE 101 42 620.8 beschrieben.

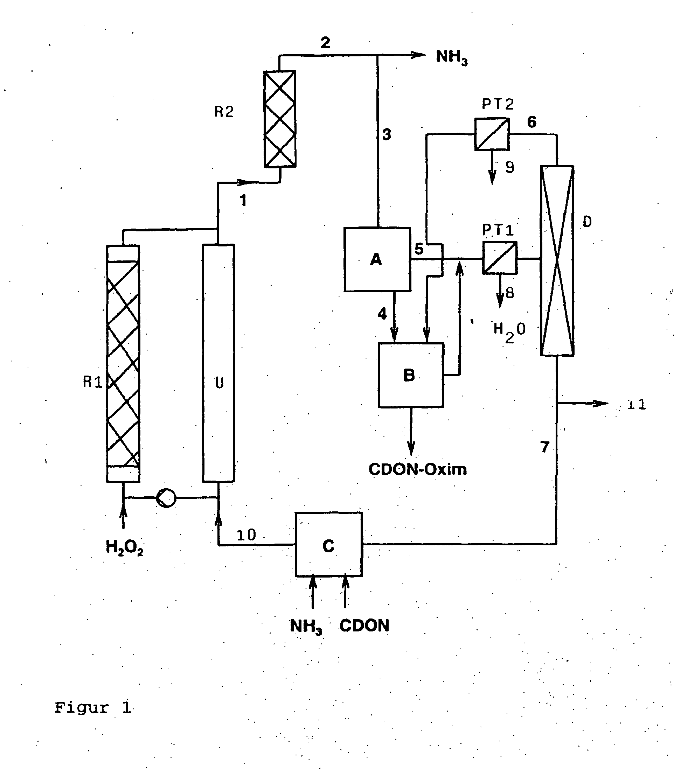

Das gesamte Verfahren wird an Hand der Figur 1, welche ein Verfahrensschema darstellt, weiter erläutert.

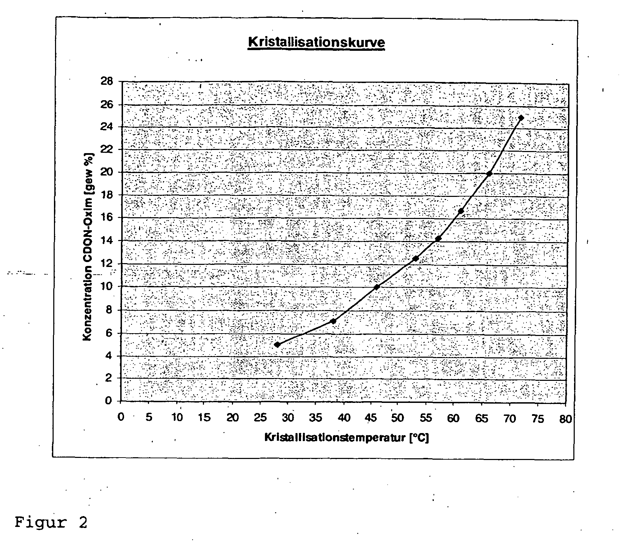

Die Figur 2 zeigt eine Kristallisationskurve von Cyclododecanonoxim (CDON-Oxim), zu dessen Herstellung sich das erfindungsgemäße Verfahren besonders eignet, in Abhängigkeit von der Temperatur, wobei n-Butanol mit 10 Gew.-% Wasser das Lösungsmittel ist.

In Figur 1, einer bevorzugten Ausführungsform der Vorrichtung, bedeuten: **R1** ein Ammoximierungsreaktor, der ein- oder mehrstufig ausgebildet sein kann. Es kann sich um einen Festbett- oder Suspensionsreaktor handeln - dargestellt ist ein Festbettreaktor. **R2** ist ein Nachreaktor zur Vervollständigung der Umsetzung und **U** eine Umlaufleitung, worin das Reaktionsgemisch auch gekühlt oder aufgeheizt werden kann. **A** steht für eine Kristallisationsvorrichtung und Trennvorrichtung zur Abtrennung des Kristallisats (= Oxim) von der Mutterlauge. **B** steht für eine Waschvorrichtung mit nachgeschalteter Fest-Flüssig-Trennung. **D** steht für eine Destillationsvorrichtung zum direkten oder azeotropen Abdestillation von Wasser. **PT1** und **PT2** bedeuten Trennvorrichtungen zur Trennung von zwei flüssigen Phasen.

Die Ammoximierung kann kontinuierlich oder diskontinuierlich bei vollständigem oder unvollständigem Ketonumsatz betrieben werden. Außer Durchlauf- und Umlaufreaktoren mit einem Katalysatorfestbett kann die Umsetzung auch in Gegenwart eines Suspensionskatalysators erfolgen. Im letzten Fall zwingend, im ersten Fall optional, schließt sich eine Fest-Flüssig-Trennung an die Umsetzung an, um Suspensionskatalysator bzw. Abrieb vom Ammoximierungsgemisch abzutrennen und den dabei erhaltenen feststofffreien Reaktoraustrag **(1)** bzw. **(2)** der Aufarbeitung zuführen zu können.

Die Reaktorausträge (1) bzw. (2) enthalten das gebildete Oxim im gewählten wässrig-organischen Lösungsmittelsystem, nicht umgesetzte Einsatzstoffe, d.h. die Carbonylverbindung, Wasserstoffperoxid und Ammoniak, und Nebenprodukte.

Der im Reaktoraustrag **2** vorhandene Ammoniak kann optional durch Druckentlastung auf Normaldruck teilweise aus der Lösung als gasförmiger Ammoniak entfernt und in einer separaten Stufe durch Kondensation aufgefangen und nach optionaler Aufreinigung in die Anlage zurück geführt werden.

Der an Ammoniak abgereicherte Reaktoraustrag **3** wird in einer Kristallisiervorrichtung (**A)** abgekühlt, wobei das entsprechende Oxim mit einer für die anschließende Beckmann-Umlagerung notwendigen Reinheit auskristallisiert. Die Kristallisiervorrichtung kann in beliebiger, dem Fachmann bekannter Weise ausgebildet sein, wobei diskontinuierliche und kontinuierliche Vorrichtungen eingesetzt werden können. Der Kristaller wird in der Regel bei Temperaturen zwischen -40°C und +60°C, vorteilhaft zwischen -10°C und +30°C betrieben, wobei sich Kühlwasser oder Kältesole als Kühlmittel eignen. Die optimale Temperatur ergibt sich aus der Löslichkeit des Oxims sowie des nicht umgesetzten Ketons im Reaktionsgemisch. In Abbildung 2 ist exemplarisch die Löslichkeit von Cyclododecanonoxim in Abhängigkeit von der Temperatur in n-Butanol mit einem Wassergehalt von 10 Gew.-% dargestellt.

Das in einer Trennvorrichtung (in Figur 1 in (A) integriert) abgetrennte Kristallisat **(4)** kann von anhaftender Mutterlauge durch Nachwaschen auf der Trennvorrichtung oder in einem separaten Wäscher **(B)** mit Alkohol beziehungsweise einem Alkohol/Wasser Gemisch befreit werden. Anschließend wird das Oxim optional getrocknet.

Es ist ein besonderer Vorteil des Verfahrens, dass auch bei unvollständigem Umsatz eines Ketons, wie Cyclododecanon, das Oxim in einer Reinheit über 99,8 % erhalten und nicht umgesetztes Keton vollständig rezykliert werden kann. Das so erhaltene Oxim kann direkt oder in einen geeigneten Lösemittel weiterverarbeitet werden , beispielsweise durch Beckmann-Umlagerung zu dem entsprechenden Lactam.

Die aus der Kristallisation mit nachfolgender Phaentrennung erhaltene und optional mit der Waschlösung aus dem Nachwaschen kombinierte Mutterlauge **(5)** enthält beim erfindungsgemäßen Verfahren im allgemeinen zwischen 0,01 Gew.-% und 5 Gew.-% Oxim, bevorzugt 0,1 Gew.-% bis 2 Gew.-% Oxim, 0,01 Gew.-% bis 10 Gew.-% nicht umgesetztes Keton, bevorzugt 0,1 Gew.-% bis 2 Gew.-% Keton, 0 Gew.-% bis 10 Gew.-% Ammoniak und 6 Gew.-% bis 20 Gew.-% Wasser. Ferner kann die Mutterlauge auch homogen gelöste Ammoniumsalze, die bei der Ammoximierung durch Weiteroxidation von Hydroxylamin gebildet und/oder gemäß DE 100103581 als Cokatalysatoren für die Ammoximierung eingesetzt wurden, in Konzentrationen von typischerweise 0,001 Mol/l bis 0,5 Mol/l, bevorzugt 0,01 Mol/l bis 0,05 Mol/l, enthalten.

Sofern die Mutterlauge bereits ein Zweiphasengemisch bildet, wird vor dem nächsten Verfahrensschritt die wässrige Phase **(8)** in einer Flüssig-Flüssig-Trennvorrichtung (PT1) abgetrennt.

Zur Reduktion des Wassergehaltes der Mutterlauge **(5)** wird diese oder die zweite Phase aus einer zwischengeschalteten Trennvorrichtung (PT1) in einer Destillationsvorrichtung mit einer oder mehreren Kolonnen (Destillationskolonne **(D)**) eingespeist und bei Temperaturen zwischen 40°C und 180°C, vorteilhaft zwischen 80°C und 140°C bei Normaldruck, Unterdruck oder einem leichten Überdruck destilliert, wobei in Abhängigkeit vom eingesetzten Lösungsmittel über Kopf im wesentlichen Wasser oder ein zweiphasiges Azeotrop aus Wasser und dem Lösungsmittel und optional Ammoniak abgetrennt wird.

Der Wassergehalt der Mutterlauge wird üblicherweise von etwa 6 Gew.-% bis 20 Gew.-% auf 0,01 Gew.-% bis 5 Gew.-%, bevorzugt auf 0,5 Gew.-% bis 2 Gew.-% reduziert, und in dieser Form als Sumpfprodukt **(7)** abgezogen.

Im Falle der Verwendung eines mit Wasser azeotropbildenden Lösemittels wird das über Kopf destillierte zweiphasige Azeotrop **(6)** nach der Kondensation in einer Phasentrennvorrichtung **(PT2)** getrennt. Die überwiegend organische Phase kann sowohl direkt als Lösemittel in die Ammoximierung zurückgefahren werden und/oder als Waschlösung für das Nachwaschen der nach der Kristallisation isolierten Kristalle verwendet werden. Die überwiegend wässrige Phase, welche noch geringe Mengen an organischem Lösungsmittel enthalten kann, wird bei Bedarf in einer weiteren Destillationsstufe entwässert oder dann verworfen.

Das Sumpfprodukt **(7)** der Destillation, welches neben dem Lösungsmittel noch Reste an Oxim, Keton, Wasser und Ammoniumsalzen sowie Spuren an weiteren Nebenprodukten und Verunreinigungen enthält, wird in einem Mischer **(C)** mit frischem Keton versetzt und das Gemisch **(10)** in die Ammoximierung zurückgefahren. Wässriges Wasserstoffperoxid wird meist an einer oder mehreren Stellen direkt in und/oder unmittelbar vor dem Reaktor eingespeist.

Bei einem kontinuierlichen Prozess mit technisch reinen Einsatzstoffen können sich im Laufe der zeit Verunreinigungen sowie Nebenprodukte aus der Ammoximierung selbst anreichern. Es erscheint deshalb vorteilhaft, einen Teilstrom des Destillationssumpfes (Purge (11)) kontinuierlich oder diskontinuierlich abzutrennen und destillativ oder extraktiv von den Verunreinigungen und Nebenprodukten zu reinigen und erst dann in die Ammoximierungsstufe zurückzuführen.

Die Struktur der als Katalysator verwendeten Formkörper für einen Festbettreaktor ist vorzugsweise zylindrisch oder sphärisch. Besonders bevorzugte Formkörper für Festbettkatalysatoren sind kugelförmig und weisen einen Partikeldurchmesser im Bereich von 0,5 bis 2,0 mm, insbesondere 0,8 bis 1,6 mm auf. Bevorzugte zylinderförmige Formkörper weisen eine größte Abmessung von 2,5 mm und ein Verhältnis von Durchmesser zu Länge im Bereich von 0,5 bis 2 auf.

Bei den titanhaltigen Katalysatoren handelt es sich insbesondere um Titansilikalit in Pulver-, Granulat-, Extrudat- oder Monolithform. Der Katalysator enthält bevorzugt Titansilikalit der allgemeinen Formel: xTiO₂(1-x)SiO₂, wobei x eine Zahl im Bereich von 0,001 bis 0,12, insbesondere größer 0,01 bis 0,1 ist.

Die Festbettkatalysatoren können ein oder mehrere Titansilikalite und bei Bedarf ein oder mehrere saure feste Cokatalysatoren enthalten. Besonders geeignete Cokatalysatoren sind Aluminiumoxid, Titandioxid, Zirkoniumdioxid, Siliciumdioxid und saure Zeolithe, wie ZSM5. Der oder die Titansilikalite sowie der oder die Cokatalysatoren können nebeneinander vorliegen oder in hintereinander geschalteten Reaktoren zur Anwendung gelangen.

Gemäß einer bevorzugten Ausführungsform enthalten die Katalysatoren sowohl Titansilikalit(e) und ein oder mehrere saure Cokatalysatoren, wobei das Gewichtsverhältnis Titansilikalit zu Cokatalysator 99:1 bis 1 zu 99 und insbesondere 9:1 bis 1:1 beträgt.

Die Herstellung von Katalysatorformkörpern erfolgt in an sich bekannter Weise, beispielsweise durch Extrudieren des Pulvergemischs in Gegenwart von Wasser und bei Bedarf eines organischen und/oder anorganischen Bindemittels mit nachfolgender Trocknung und Kalzinierung. Beispielhaft wird auf die EP 1 138 387 A1 verweisen.

Gemäß einer bevorzugten Ausführungsform des erfindungsgemäßen Verfahrens findet die Ammoximierung in Gegenwart eines zumindest teilweise im Reaktionsgemisch löslichen Ammoniumsalzes statt, wobei das Ammoniumsalz zugesetzt oder in-situ aus dem anwesenden Ammoniak und einer zugesetzten Säure gebildet worden sein kann. Als nicht beschränkende Beispiele für Ammoniumsalze seien hier genannt: Ammoniumnitrit, Ammoniumnitrat, Ammoniumsalze von Carbonsäuren, wie insbesondere Ammoniumacetat, Hydroxylammoniumnitrit, Hydroxylammoniumnitrat, Hydroxylammoniumacetat, Mono-, Di und Triammoniumphosphat, Mono- und Diammoniumpyrophosphat, Mono- und Diammonium-1-hydroxyethan-1,1-diphosphonsäure sowie Mono- und Diammoniumstannat. Es ist ein Vorteil dieser Erfindung, dass diese Ammoniumsalze mit der Mutterlauge problemlos in einem Kreis gefahren und nicht separat aufgearbeitet und erneut zugesetzt werden müssen.

Die Reaktionsbedingungen zur Durchführung der Ammoximierung können in weiten Grenzen liegen und sind dem Stand der Technik zu entnehmen. Wasserstoffperoxid kann bezüglich der Carbonylverbindung im Unter- oder Überschuss verwendet werden; bevorzugt liegt das Molverhältnis im Bereich von 0,5 bis 1,5. Ammoniak wird in der Regel im Überschuss gegenüber Wasserstoffperoxid sowie im Überschuss gegenüber der Carbonylverbindung eingesetzt. Zweckmäßigerweise liegt das Molverhältnis von Ammoniak zu Wasserstoffperoxid im Bereich von 1,5 bis 3. Die Ammoximierung erfolgt bei einer Temperatur im Bereich von 25 bis 150°C, insbesondere bei 50 bis 125°C, insbesondere bevorzugt bei 60 bis 110°C. Üblicherweise wird die Umsetzung unter dem sich bei der gewählten Temperatur einstellenden Druck durchgeführt - bei Bedarf kann der Druck durch die Anwesenheit eines Inertgases, wie Stickstoff, erhöht werden. Meistens liegt der Druck im Bereich von 1 - 15 bar (0,1 bis 1,5 MPa).

Bei der Verwendung eines Festbettkatalysators wird das Reaktionsgemisch in Rieselbettfahrweise oder in Blasenfahrweise, wobei letztere bevorzugt wird, durch den oder die Reaktoren geleitet. Vorzugsweise wird das Reaktionsgemisch bei einem Umsatz der Carbonylverbindung von mindestens 80 %, vorzugsweise größer 90 % aus der Ammoximierungsstufe abgezogen und der Aufarbeitung zugeführt.

Durch das erfindungsgemäße Verfahren unter Verwendung des besonders bevorzugten n-Butanol als Lösungsmittel, ist es möglich geworden, in sehr einfacher Weise auch Ketone mit gleich oder mehr als 6 C-Atomen, insbesondere 8 - 12 C-Atomen, in hoher Reinheit und Ausbeute und mit hoher Peroxid-Selektivität zu ammoximieren und Wasser in wirtschaftlicher Weise aus dem Prozess auszuschleusen. Das Verfahren eignet sich insbesondere für einen kontinuierlichen und störungsarmen Betrieb. In besonderer Weise eignet sich das Verfahren zur kontinuierlichen Herstellung von Cyclododecanonoxim.

Die Erfindung wird anhand der nachfolgenden Beispiele, welche keinesfalls limitierend sein sollen, weiter verdeutlicht.

### Beispiele:

### Beispiel 1: Ammoximierung

Ein Kreislaufreaktor mit integriertem Festbett und Überlauf (Gesamtvolumen 530 ml) wurde mit 50 g zylindrischen Katalysatorformkörpern - Extrudate mit 3 mm Durchmesser und 2 mm Dicke aus Titansilikalit TS-1 mit 20 Gew.-% Aluminiumoxid und hergestellt gemäß EP 1 138 387 - und 50 g Glaskugeln befüllt. Im Reaktor wurden 350 g einer n-butanolischen Lösung von 87,5 g Cyclododecanon (CDON), 3,2 g Ammoniumacetat, 7,0 g Wasser und 3,5 g Diglyme als interner Standard vorgelegt und auf 80°C temperiert. Durch Aufdrücken von Stickstoff wurde ein Druck von 12 bar (1,2 MPa) eingestellt. Über eine Dosierpumpe wurden anschließend 9,5 g/h Ammoniak eingespeist; nach 15 Minuten wurden zusätzlich über eine weiter Dosierpumpe 11,2 g/h Wasserstoffperoxid (50,0 Gew.-%) zugegeben.

Nach 3 Stunden wurde durch Beginn der Einspeisung von 102,0 g/h einer 25 Gew.-%-igen Cyclododecanon Lösung in n-Butanol auf kontinuierliche Fahrweise umgestellt.

Die überlaufende Reaktionslösung wurde in einem Abscheider aufgefangen. Der CDON-Umsatz wurde während der Reaktion per Gaschromatographie verfolgt und der Wasserstoffperoxidgehalt iodometrisch bestimmt. Innerhalb von 20 Stunden betrug der CDON-Umsatz ca. 87,9 % bei einer Peroxidselektivität bezogen auf umgesetztes CDON von ca. 74,7 %.

### Beispiel 2: Ammoximierung

Der Versuch wurde entsprechend Beispiel 1 durchgeführt.

Der Festbettreaktor wurde mit 50 g Katalysatorformkörpern - sphärische agglomerierte Formkörper mit einem Durchmesser von 2,5 - 3,0 mm und hergestellt aus Titansilikalit TS-1 mit 20 Gew.-% Aluminiumoxid - und 50 g Glaskugeln befüllt und die Umsetzung 20 Stunden betrieben. Der CDON-Umsatz 87,7 % betrug bei einer Peroxidselektivität, bezogen auf umgesetztes CDON, von 72,8 %.

### Beispiel 3: Ammoximierung

Der Versuch wurde entsprechend Beispiel 1 durchgeführt.

Der Festbettreaktor wurde mit 50 g Katalysatorformkörpern - sphärische agglomerierte Formkörper mit einem Durchmesser von 1,0 - 1,4 mm (hergestellt aus Titansilikalit TS-1 mit 20 Gew.-% Aluminiumoxid) - und 50 g Glaskugeln befüllt. Der kontinuierliche Betrieb wurde 20 Stunden aufrecht erhalten:
Der CDON-Umsatz betrug 93,2 % bei einer Peroxidselektivität, bezogen auf umgesetztes CDON, von 78,4 %.

### Beispiel 4: Ammoximierung

Die Einspeisungen aus Beispiel 3 wurden variiert.

Es wurden 7,0 g/h Ammoniak, 8,2 g/h Wasserstoffperoxid (50,0 Gew.-%) und 73,0 g/h einer n-butanolischen Lösung von 18,2 g Cyclododecanon, 0,33 g Ammoniumacetat, 1,45 g Wasser und 0,73 g Diglyme als interner Standard eingespeist. Innerhalb 70 Stunden kontinuierlichen Betriebs betrug der CDON-Umsatz 95,3 % bei einer Peroxidselektivität, bezogen auf umgesetztes CDON, von 81,2 %.

### Beispiel 5: Kristallisation

260 g der Reaktionsmischung aus Beispiel 4 wurde aus dem Abscheider abgelassen, wobei beim Entspannen ein Großteil des Ammoniakgases entwich, welches über eine Kühlfalle kondensiert und in den Prozess zurückgeführt wurde. Die Reaktionsmischung mit einem Wassergehalt von 10,5 Gew.-% wurde auf 0°C abgekühlt und der nach 3 Stunden ausgefallene Niederschlag über eine Nutsche abgetrennt. Der Niederschlag wurde mit wenig kaltem n-Butanol nachgewaschen und getrocknet. Es wurden 44,9 g Oxim isoliert. Beim Stehenlassen der Mutterlauge kristallisierten weitere 4,5 g Oxim aus.

Die Gesamtausbeute an Oxim betrug 49,4 g (88,2 % d. Th.) in einer Reinheit >99,85%

Nach Vereinigung der Mutterlauge und der Waschlösung enthielt die wässrige n-Butanol-Lösung noch ca. 2,7 g Cyclododecanon, 1,0 g Ammoniumacetat, Spuren von Ammoniak und 5,9 g Oxim und ca. 10 Gew.-% Wasser.

### Beispiel 6: Abtrennung von Wasser aus der Mutterlauge

Aus 280 g der in Beispiel 5 erhaltenen n-butanolischen Mutterlaugenlösung wurden destillativ 67 g eines zweiphasigen Azeotrops abgetrennt. Der Wassergehalt wurde hierbei von ca. 10 Gew.-% in der Mutterlauge auf ca. 1,4 Gew.-% im Sumpfgemisch reduziert. Nach der Destillation enthielt das Sumpfgemisch neben n-Butanol noch ca. 2,7 g Cyclododecanon, 1,0 g Ammoniumacetat und 5,9 g Oxim. Das Sumpfgemisch wurde nach Zugabe von Cyclododecanon in die Ammoximierungsstufe zurückgeführt - Umsatz und Peroxidausbeute blieben im wesentlichen erhalten.

## Patentansprüche

1. Verfahren zur Herstellung von Oximen durch Ammonoximierung von Carbonylverbindungen, umfassend
(i) Umsetzung einer Carbonylverbindung mit 6 bis 20 C-Atomen mit Ammoniak und Wasserstoffperoxid in Gegenwart eines zumindest teilweise wasserlöslichen organischen Lösungsmittels und eines titanhaltigen heterogenen Katalysators,
(ii) Abtrennung des Katalysators vom Reaktionsgemisch,
(iii) Auskristallisation des Oxims und Abtrennung desselben von der Mutterlauge,
(iv) Ausschleusung von Wasser aus der Mutterlauge und
(v) Rückführung des Lösungsmittels in Stufe (i),
**dadurch gekennzeichnet,**
**dass** man in der Stufe (i) ein unter Ammoximierungsbedingungen stabiles Lösungsmittel verwendet, das einen Siedepunkt größer 100°C aufweist und/oder mit Wasser ein zweiphasiges Azeotrop bildet, aus der Mutterlauge Wasser oder ein wasserhaltiges zweiphasiges Azeotrop abdestilliert und den Destillationssumpf in die Stufe (i) zurückführt.

2. Verfahren nach Anspruch 1,
**dadurch gekennzeichnet,**
**dass** man die überwiegend organische Phase des Azeotrops in die Stufe (i) zurückführt.

3. Verfahren nach Anspruch 1 oder 2,
**dadurch gekennzeichnet,**
**dass** man als Lösungsmittel einen Alkohol mit 2 bis 6 C-Atomen verwendet, insbesondere einen einwertigen Alkohol mit 4 bis 6 C-Atomen oder einem zweiwertigen Alkohol.

4. Verfahren nach einem der Ansprüche 1 bis 3,
**dadurch gekennzeichnet,**
**dass** man als Lösungsmittel n-Butanol verwendet.

5. Verfahren nach einem der Ansprüche 1 bis 4,
**dadurch gekennzeichnet,**
**dass** man das Oxim bei einer Temperatur im Bereich von -40 bis +60 °C, insbesondere -10 bis +30 °C auskristallisieren lässt.

6. Verfahren nach einem der Ansprüche 1 bis 5,
**dadurch gekennzeichnet,**
**dass** man ein lineares oder cyclisches Keton mit 6 bis 12 C-Atomen, insbesondere 8 bis 12 C-Atomen, in Gegenwart von n-Butanol als Lösungsmittel ammoximiert.

7. Verfahren nach einem der Ansprüche 1 bis 6,
**dadurch gekennzeichnet,**
**dass** man die Ammoximierung in Gegenwart eines Titansilikalit enthaltenden Katalysators durchführt.

8. Verfahren nach einem der Ansprüche 1 bis 7,
**dadurch gekennzeichnet,**
**dass** man die Ammoximierung zusätzlich in Gegenwart eines festen sauren Cokatalysators, insbesondere aus der Reihe A₂O₃, TiO₂, ZrO₂ und sauren Zeolithe und/oder eines löslichen Ammoniumsalzes, insbesondere eines Salzes aus der Reihe Ammoniumnitrat, Hydroxylammoniumnitrat, Ammoniumphosphate, Ammoniumpyrophosphate, Ammoniumsalze von Carbonsäuren und Ammoniumstannaten, durchführt.

9. Verfahren nach einem der Ansprüche 1 bis 8,
**dadurch gekennzeichnet,**
**dass** man die Ammoximierung kontinuierlich in einem oder mehreren hintereinander geschalteten Festbettreaktoren in Rieselbett- oder Blasenfahrweise bei 25 bis 150°C, insbesondere 50 bis 125°C, durchführt.

## Claims

1. A method for synthesis of oximes by ammoximation of carbonyl compounds, comprising
(i) reacting a carbonyl compound having 6 to 20 carbon atoms with ammonia and hydrogen peroxide in the presence of an at least partially watersoluble organic solvent and of a titanium-containing heterogeneous catalyst,
(ii) separating the catalyst from the reaction mixture,
(iii) crystallizing the oxime and separating same from the mother liquor
(iv) removing water from the mother liquor, and
(v) returning the solvent into stage (i),
**characterized in that** stage (i) utilizes a solvent which is stable under ammoximation conditions and which has a boiling point above 100°C and/or forms a two-phase azeotrope with water; water or a water-containing two-phase azeotrope is distilled off from the mother liquor; and the distillation bottoms are recycled to stage (i).

2. A method according to claim 1, **characterized in that** the predominantly organic phase of the azeotrope is recycled to stage (i).

3. A method according to claim 1 or 2, **characterized in that** the solvent used is an alcohol having 2 to 6 carbon atoms, in particular a monohydric alcohol having 4 to 6 carbon atoms or a dihydric alcohol.

4. A method according to any one of claims 1 to 3, **characterized in that** n-butanol is used as solvent.

5. A method according to any one of claims 1 to 4, **characterized in that** the oxime is allowed to crystallize at a temperature in the range from -40 to +60°C, in particular -10 to +30°C.

6. A method according to any one of claims 1 to 5, **characterized in that** a linear or cyclic ketone having 6 to 12 carbon atoms, in particular 8 to 12 carbon atoms, is ammoximated in the presence of n-butanol as solvent.

7. A method according to any one of claims 1 to 6, **characterized in that** the ammoximation is carried out in the presence of a catalyst containing titanium silicalite.

8. A method according to any one of claims 1 to 7, **characterized in that** the ammoximation is carried out in the additional presence of a solid acidic cocatalyst, in particular from the group consisting of Al₂O₃, TiO₂, ZrO₂ and acidic zeolites, and/or of a soluble ammonium salt, in particular a salt from the group consisting of ammonium nitrate, hydroxylammonium nitrate, ammonium phosphates, ammonium pyrophosphates, ammonium salts of carboxylic acids and ammonium stannates.

9. A method according to any one of claims 1 to 8, **characterized in that** the ammoximation is carried out continuously in one or more fixed-bed reactors, connected in series, in trickle-bed or bubble-flow mode at 25 to 150°C, in particular 50 to 125°C.

## Revendications

1. Procédé pour la préparation d'oximes par ammoximation de composés carbonyle, comprenant
(i) la transformation d'un composé carbonyle comprenant 6 à 20 atomes de carbone avec de l'ammoniac et du peroxyde d'hydrogène en présence d'un solvant organique au moins partiellement soluble dans l'eau et d'un catalyseur hétérogène contenant du titane,
(ii) séparation du catalyseur du mélange réactionnel,
(iii) séparation par cristallisation de l'oxime et séparation de celle-ci de la lessive-mère,
(iv) évacuation de l'eau de la lessive-mère et
(v) recyclage du solvant dans l'étape (i),
**caractérisé en ce qu'**on utilise dans l'étape (i) un solvant stable dans les conditions d'ammoximation qui présente un point d'ébullition supérieur à 100°C et/ou qui forme avec l'eau un azéotrope à deux phases, on élimine l'eau ou un azéotrope à deux phases contenant de l'eau par distillation de la lessive-mère et on recycle le fond de distillation dans l'étape (i).

2. Procédé selon la revendication 1, **caractérisé en ce qu'**on recycle la phase principalement organique de l'azéotrope dans l'étape (i).

3. Procédé selon la revendication 1 ou 2, **caractérisé en ce qu'**on utilise comme solvant un alcool comprenant 2 à 6 atomes de carbone, en particulier un alcool monohydrique comprenant 4 à 6 atomes de carbone ou un alcool dihydrique.

4. Procédé selon l'une quelconque des revendications 1 à 3, **caractérisé en ce qu'**on utilise comme solvant du n-butanol.

5. Procédé selon l'une quelconque des revendications 1 à 4, **caractérisé en ce qu'**on laisse l'oxime se séparer par cristallisation à une température dans la plage de -40 à +60°C, en particulier de -10 à +30°C.

6. Procédé selon l'une quelconque des revendications 1 à 5, **caractérisé en ce qu'**on ammoxime une cétone linéaire ou cyclique comprenant 6 à 12 atomes de carbone, en particulier 8 à 12 atomes de carbone, en présence de n-butanol comme solvant.

7. Procédé selon l'une quelconque des revendications 1 à 6, **caractérisé en ce qu'**on réalise l'ammoximation en présence d'un catalyseur contenant du silicalite de titane.

8. Procédé selon l'une quelconque des revendications 1 à 7, **caractérisé en ce qu'**on réalise l'ammoximation en outre en présence d'un cocatalyseur acide solide, en particulier de la série Al₂O₃, TiO₂, ZrO₂ et des zéolithes acides et/ou d'un sel d'ammonium soluble, en particulier d'un sel de la série nitrate d'ammonium, nitrate d'hydroxylammonium, phosphates d'ammonium, pyrophosphates d'ammonium, sels d'ammonium d'acides carboxyliques et stannates d'ammonium.

9. Procédé selon l'une quelconque des revendications 1 à 8, **caractérisé en ce qu'**on réalise l'ammoximation en continu dans un ou plusieurs réacteurs à lit fixe disposés les uns derrière les autres, dans un mode opératoire à bulles ou en lit à ruissellement de 25 jusqu'à 150°C, en particulier de 50 jusqu'à 125°C.
